# EUROPÄISCHE PATENTSCHRIFT

(11) **EP 2 162 172 B1**
(45) Veröffentlichungstag und Bekanntmachung des Hinweises auf die Patenterteilung: **30.05.2018**
(21) Anmeldenummer: 08773533.8
(22) Anmeldetag: 19.06.2008
(51) Int. Cl.: A61M 5/30, A61M 5/20, A61M 5/32

(54) **EINWEGINJEKTOR MIT MINDESTENS EINEM DRUCKSTAB UND EINER VERSCHLUSSKAPPE**
DISPOSABLE INJECTOR WITH AT LEAST ONE COMPRESSION BAR AND A CLOSURE CAP
INSTRUMENT D'INJECTION À USAGE UNIQUE COMPRENANT AU MOINS UNE BARRE DE PRESSION ET UN CAPUCHON DE FERMETURE

(30) Priorität: 06.07.2007 DE 102007031714
(43) Veröffentlichungstag der Anmeldung: 17.03.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Thämer, Wolfgang
(86) Internationale Anmeldenummer: PCT/EP2008/004949
(87) Internationale Veröffentlichungsnummer: WO 2009/006986

(56) Entgegenhaltungen:
- EP-A- 1 336 419
- WO-A-94/21316
- WO-A-2005/044344
- WO-A-2005/056077
- WO-A-2007/073839
- US-A- 4 227 528
- US-A- 4 378 015

## Beschreibung

Die Erfindung ist in Anspruch 1 definiert und betrifft einen Einweginjektor mit einem Gehäuse, in dem oder an dem - jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst und wobei zumindest ein einen Stempelteller bildender Teil des Kolbenbetätigungsstempels zwischen dem Federenergiespeicher und dem Kolben der Zylinder-Kolben-Einheit positioniert ist, wobei das Gehäuse mindestens einen Druckhaken aufweist, der jeweils im Bereich seines freien Endes mindestens eine Abstützfläche hat, wobei die sperrende Lage des Druckhakens durch ein in einer Sperrstellung positioniertes Auslöseelement gesichert ist und wobei das Auslöseelement eine Sperrstellung hat, in der es an einer Verschlusskappe gesichert anliegt.

Aus der DE 36 44 984 A1 ist u.a. ein Injektor bekannt. Er hat einen federvorgespannten Kolbenbetätigungsstempel, dessen rückwärtige Stempelstange an ihrem freien Ende elastische Zughaken aufweist. Die Zughaken halten den Kolbenbetätigungsstempel formschlüssig an einer Kante des Injektorgehäuses fest. Sie haben hierzu nur eine geringe Auflagefläche am Gehäuse. Zum Auslösen des Injektors werden die Zughaken von der sie haltenden Kante geschoben. In der Folge schießt der federvorgespannte Kolbenbetätigungsstempel nach vorn, um eine Injektion durchzuführen.

Die WO 2005/044344 A1 und die EP 1 336 419 A1 beschreiben jeweils einen Nadel-Injektor mit einer Nadelschutzvorrichtung, dessen Kolbenbetätigungsstempel ein Zugstab bzw. eine Zughülse ist. Der Kolbenbetätigungsstempel, der von einer bei der Injektion den Kolben antreibender Schraubendruckfeder auf Zug belastet wird, stützt sich an gehäuseseitigen Stützelementen ab. Dabei liegen die Stützelemente am kleinsten Durchmesser des Kolbenbetätigungsstempels an, wodurch sich in der Kontaktzone eine besonders große Flächenpressung ergibt. Die WO9503844 offenbart einen Einweginjektor mit einer Klinke, die in eine Nut im Kolbenbetätigungsstempel einhakt.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruchs gelöst. Dazu liegt an der Abstützfläche der Kolbenbetätigungsstempel mit seinem zumindest bereichsweise als zylindrische Scheibe ausgebildeten Stempelteller. Die Verschlusskappe umgibt den unteren - mit einer Düse ausgestatteten - Bereich des Zylinders der Zylinder-Kolben-Einheit Außerdem hat das Auslöseelement eine Lösestellung, die ein seitliches Zurückweichen des Druckhakens - unter Freigabe des Kolbenbetätigungsstempels - bewirkt, sodass der Kolbenbetätigungsstempel ungehindert nach unten schnellt.

Mit der Erfindung wird hier beispielsweise ein nadelfreier Einmalinjektor vorgestellt, dessen Kolbenbetätigungsstempel bei einem Auslösevorgang des Einweginjektors freigegeben wird. Dazu wird zum Vorspannen und Halten des Federenergiespeichers der Kolbenbetätigungsstempel über mindestens einen, am Gehäuse angeordneten oder im Gehäuse integrierten, Druckstab form- und kraftschlüssig gehalten. Der oder die Druckstäbe werden von einem Auslöseelement bis zum Gebrauch des Einweginjektors in ihrer Sperrposition gehalten und mittels einer Verschlusskappe, die zugleich die Zylinder-Kolben-Einheit steril verschließt, gesichert. Zum Auslösen des Injektors werden der oder die Druckstäbe freigegeben, so dass sich der Kolbenbetätigungsstempel - unter der Wirkung des Federenergiespeichers - zumindest annähernd parallel zur Mittellinie des Einweginjektors bewegen kann.

Weitere Einzelheiten der Erfindung ergeben sich aus den Unteransprüchen und den nachfolgenden Beschreibungen schematisch dargestellter Ausführungsbeispiele.
- Figur 1:: Einweginjektor mit zwei Druckstäben und konischer Bundfläche;
- Figur 2:: wie Figur 1, jedoch entsichert und betätigt;
- Figur 3:: wie Figur 2, jedoch nach dem Medikamentenausstoß;
- Figur 4:: Einweginjektor mit zwei Stützstäben und planer Stirnfläche;
- Figur 5:: Einweginjektor mit zwei in Sperrstellung verformten Druckstäben und zusätzlich geführtem Kolbenbetätigungsstempel;
- Figur 6:: wie Figur 5, jedoch entsichert und betätigt (fiktiver Zustand);
- Figur 7:: wie Figur 6, jedoch mit entleertem Zylinder;
- Figur 8:: Dimetrische Ansicht zu Figur 5;
- Figur 9:: Einweginjektor mit zwei in Sperrstellung verformten Druckstäben und zusätzlicher Sicherung;
- Figur 10:: Ausschnittvergrößerung zu Figur 9;
- Figur 11:: Ausschnittvergrößerung zu Figur 9, jedoch um 90 Winkelgrade versetzt;
- Figur 12:: wie Figur 9, jedoch durch Entfernen der Banderole entsichert und betätigt (fiktiver Zustand);
- Figur 13:: wie Figur 12, jedoch mit entleertem Zylinder;
- Figur 14:: Dimetrische Ansicht zu Figur 9.

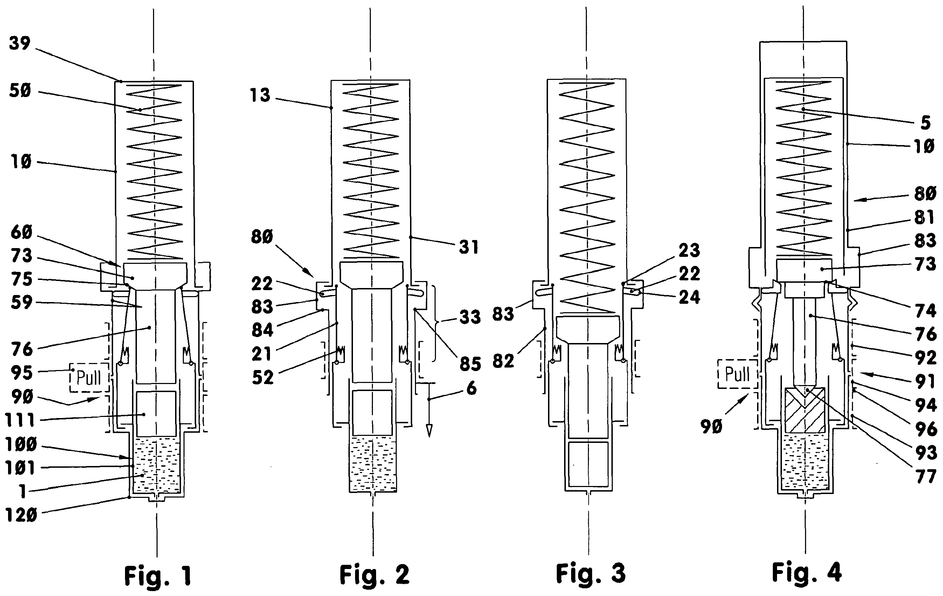

Die Figuren 1 bis 3 zeigen eine vereinfachte Prinzipskizze eines Einweginjektor-Typs mit einem dauergeladenen Federenergiespeicher in drei verschiedenen Auslösezuständen. Der gezeigte Einweginjektor besteht aus einem Gehäuse (10), einer z.B. mit einer Injektionslösung vorbefüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (90) angeordnet. Die Zylinder-Kolben-Einheit (100) ist vorn mit einer Verschlusskappe (120) verschlossen.

Das Gehäuse (10) ist ein topfförmiger, unten offener Hohlkörper mit obenliegendem Boden (39). Im mittleren Bereich, dem Mantelbereich (31), hat das Gehäuse (10) z.B. zwei einander gegenüberliegende fensterartige Durchbrüche (33), vgl. Figur 2. Am unteren Rand des einzelnen Durchbruches (33) ist jeweils ein Druckstab (21) gelenkig gelagert.

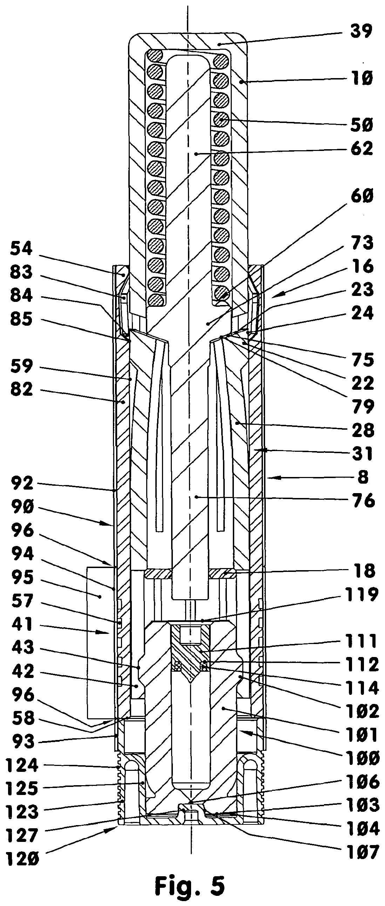

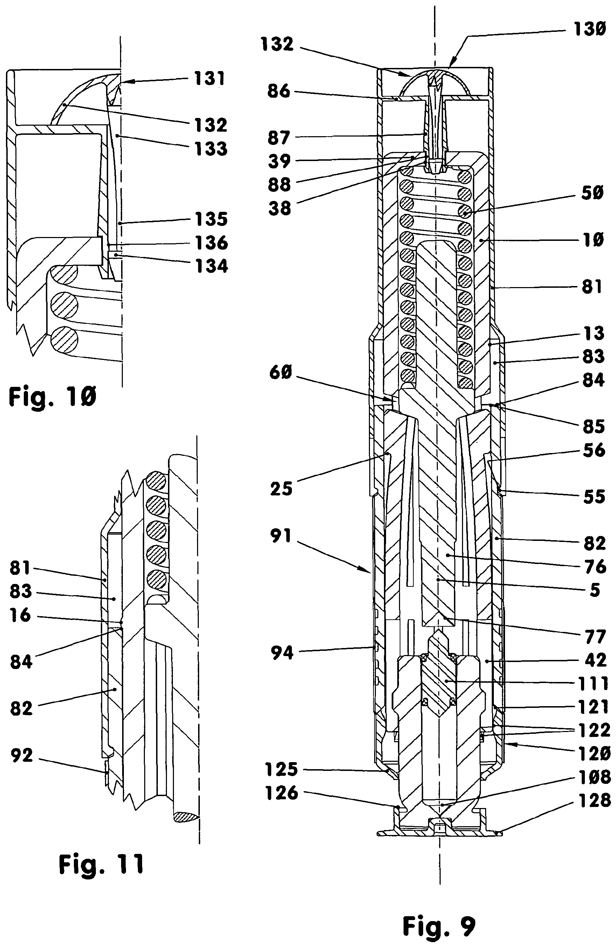

Die Druckstäbe (21) sind hier nur beispielhaft in Schwenkgelenken angeordnet und über Federelemente (52) am Gehäuse (10) abgestützt. Die Federelemente (52) drücken die Stützstäbe (21) zumindest annähernd radial nach außen gegen das Auslöseelement (82), vgl. Figur 1 bis 3. Dort liegen sie über Nocken (22) am Auslöseelement (82) an. Die Nocken (22) können dabei z.B. auch 5 bis 20 Millimeter unterhalb des jeweiligen freien oberen Endes der Druckstäbe (21) liegen. Sind die Druckstäbe (21) am Gehäuse (10) angeformt, vgl. Figuren 5 und 9, so federn sie als elastische Biegebalken (28) nach außen.

Die beiden auf Druck belasteten Druckstäbe (21) halten den Kolbenbetätigungsstempel (60) an dessen Stempelteller (73) in seiner vorgespannten Lage, vgl. Figur 1. Dazu stützen sich die Druckstäbe (21) mit ihren Abstützflächen (23) am Stempelteller (73) ab. Die Größe der jeweiligen Kontaktfläche zwischen einer Abstützfläche (23) und der entsprechenden Stelle am Stempelteller (73) liegt im Bereich von 2 bis 20 mm².

Auf der der Mittellinie (5) abgewandten Seite weist jeder Druckstab (21) an seinem Nocken (22) eine Anlagefläche (24) auf.

Im unteren Bereich des Gehäuses (10) befinden sich Halteelemente zur Befestigung der Zylinder-Kolben-Einheit (100).

Die Zylinder-Kolben-Einheit (100) besteht im Ausführungsbeispiel aus einem, mit einer Injektionslösung (1) befüllten, transparenten Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Nach Figur 1 ist die untere Hälfte des Gehäuses (10) von dem hülsenartigen Auslöseelement (82) umgeben. Das Auslöseelement (82) ist auf der radialen Außenfläche (13) des Gehäuses (10) längsverschiebbar gelagert. Es hat im oberen Bereich auf der Höhe der Nocken (22) eine umlaufende Aufweitung (83). Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Druckstäbe (21) mit ihren Nocken (22) aufnehmen kann. Die Innenkontur der Aufweitung (83) ist z.B. ein Kanal mit einer Rücksprungflanke (84), die hier eine zur Mittellinie (5) des Injektors normale Ebene darstellt. Der Übergang zwischen der beispielsweise zylindrischen Innenwandung des Auslöseelements (82) und der Rücksprungflanke (84) ist z.B. als scharfkantige Kante (85) ausgebildet. Nach Figur 1 liegen die Nocken (22) mit ihren außen liegenden Anlageflächen (24) an der Innenwandung (59) des Auslöseelements (82) sichernd an.

Der im Gehäuse (10) angeordnete Kolbenbetätigungsstempel (60) ist hier in zwei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der obere Bereich, der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite (74) weist eine um den Kolbenschieber (76) herum angeordnete Bundfläche (75) auf. Sie hat die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Federkraft wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

An das untere Ende des Auslöseelements (82) schließt sich die Verschlusskappe (120) an. Letztere umhüllt steril den unteren Teil der Zylinder-Kolben-Einheit (100). Hier ist die Verschlusskappe (120) auf dem unteren Bereich des Gehäuses (10) gelagert. Die Verschlusskappe (120) und das hülsenartige Auslöseelement (82) sind zumindest bereichsweise mit einem Klebeetikett (91) ummantelt, vgl. auch Figur 4. Das Klebeetikett (91) besteht aus einem Hauptteil (92), einer Abreißbanderole (94) und einem Kappenteil (93). Die Abreißbanderole (94) ist mit den Etikettteilen (92, 93) durch eine Sollbruchstelle (96), z.B. eine Perforation oder eine durchgehende Materialdünnstelle, verbunden. Die Abreißbanderole (94) ist hierbei über der zwischen dem Auslöseelement (82) und der Verschlusskappe (120) gelegenen Montagefuge angeordnet. Sie endet in einer abstehenden Abreißfahne (95).

Es kann auch ein vollflächiges Klebeetikett verwendet werden, das im Bereich der die Teile (82) und (120) trennenden Montagefuge ein reißfestes Zugmittel enthält. Das Zugmittel, z.B. ein Faden, ein Kunststoffstreifen, ein dünner Draht oder dergleichen steht an einer Seite über das Etikett über. Beim Abziehen des Zugmittels wird das Etikett im Bereich der Montagefuge gezielt aufgetrennt.

Zum Entsichern des Injektors wird die Abreißbanderole (94) abgezogen, so dass die Klebeverbindung zwischen der Verschlusskappe (120) und dem Auslöseelement (82) aufgehoben ist. Zum anschließenden Betätigen des Einweginjektors wird - nach dem Entfernen der Verschlusskappe (120) der Zylinder-Kolben-Einheit (100) - der Einweg-Injektor auf der Injektionsstelle positioniert. Nun kann das Auslöseelement (82) in Richtung der Zylinder-Kolben-Einheit (100) verschoben werden. Bei diesem Vorgang gleitet das Auslöseelement (82) auf der Außenwandung (13) des Gehäuses (10) linear nach unten, also in Richtung der Injektionsstelle. Die Anlageflächen (24) der Druckstäbe (21) rutschen über die Kante (85) und springen unter der Kraft des Federelements (50) entsichernd radial nach außen in die Aufweitung (83). Der Kolbenbetätigungsstempel (60) schnellt ungehindert nach unten, vgl. Figur 3. Der Zylinder (100) wird entleert. In einem Beispiel, das nicht Teil der Erfindung ist, kann anstelle einer linearen Gleitbewegung des Auslöseelements (82) auf dem Gehäuse (10) auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Die Figur 4 zeigt eine Variante mit einer geänderten Auslöseeinheit (80) und einem anderen Kolbenschieber (76). Am Auslöseelement (82) ist eine Auslösekappe (81) befestigt, die das hintere Ende des Gehäuses (10) vollständig umgibt. Hierbei umfasst die Auslösekappe (81) die Aufweitung (83).

Bei dieser Variante ist die Bundfläche (74) des Stempeltellers (73) plan ausgeführt. Die Bundfläche (74) ist normal zur Mittellinie (5) orientiert. Sie kontaktiert über eine abgerundete Kante die oberen Stirnflächen der Druckstäbe (21). Diese Stirnflächen sind keilförmig, kegelstumpfmantelförmig oder sphärisch gekrümmt. Die Krümmung ist jeweils so orientiert, dass auf die Druckstäbe (21) - wie bei der Variante nach den Figuren 1 bis 3 - eine radial nach außen wirkende Kraft auftritt.

Des Weiteren verfügt der Kolbenschieber (76) über eine zentrale kegelförmige Spitze (77). Diese Spitze (77) ragt in eine entsprechende Ausnehmung des Kolbens (111) hinein. Auf diese Weise kann der Kolben (111) den bewegten Kolbenbetätigungsstempel (60) oder Teile davon zusätzlich zentrieren und führen.

Die Figuren 5 bis 8 zeigen eine Ausführungsform des in den Figuren 1 bis 3 beschriebenen Prinzips. Hier ist das tragende Bauteil ein einteiliges Gehäuse (10). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

Der im wesentlichen rohrförmige Mantelbereich (31) ist oben durch einen z.B. ebenen Boden (39) verschlossen. In der unteren Hälfte des Mantelbereichs (31) befindet sich zwei einander gegenüberliegende, angeformte Druckstäbe (21). Die Anformstelle für die Druckstäbe (21) liegt knapp oberhalb des Fixierbereichs (41). Zur Ausbildung des jeweiligen Druckstabs (21) befindet sich im Mantelabschnitt (31) ein schmaler, zumindest annähernd u-förmiger Spalt, der den einzelnen Druckstab seitlich und oben umgibt. Der Druckstab (21) hat auf ca. 80% seiner Länge die Wandstärke und die Krümmung der Wandung des Gehäuses (10). Dieser Bereich hat u.a. auch die Funktion eines federelastischen Biegebalkens (28). Er hat einen sichelförmigen Querschnitt.

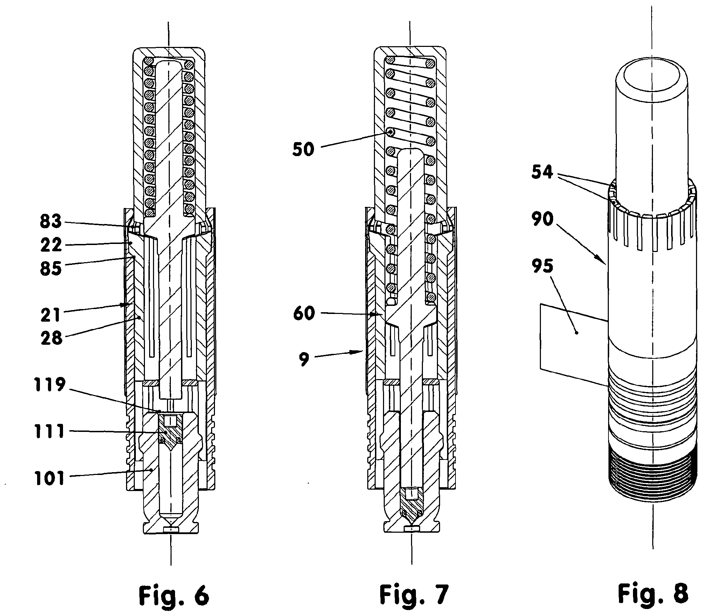

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 6 und 7 ist der Druckstab (21) im unverformten Zustand dargestellt.

Das hier obere freie Ende des einzelnen Druckstabs (21) wird durch den radial nach außen abstehenden Nocken (22) gebildet. Letzterer hat zumindest eine Abstützfläche (23) und eine Anlagefläche (24). Nach Figur 5 liegt auf der Abstützfläche (23) der Stempelteller (73) des gespannten Einweg-Injektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Druckstäbe (21) oder die Bundfläche (75) zumindest im Kontaktbereich eine keramische Panzerung. Im Ausführungsbeispiel nach Figur 5 ist die Bundfläche (75) durch eine z.B. aufgeklebte kegelstumpfmantelförmige Unterlagscheibe (79) verstärkt.

Die Anlagefläche (24) der Nocken (22) ist Teil eines Zylindermantels, dessen Durchmesser z.B. 3 bis 4 Millimeter größer als der Außendurchmesser des Gehäuses (10) ist. Die Anlagefläche (24) kontaktiert bei gespanntem Einweginjektor die Innenwandung (59) des hülsenartigen Auslöseeelements (82). Ggf. hat - zur Minimierung der Flächenpressung - die Anlagefläche (24) eine Krümmung, die der Innenwandung (59) entspricht.

Unterhalb des Mantelabschnitts (31) befindet sich der Fixierbereich (41) zur Aufnahme der einbaubaren Zylinder-Kolben-Einheit (100). Der Fixierbereich (41) umfasst z.B. acht parallel zur Mittellinie (5) ausgerichtete Federhaken (42). Die Federhaken (42) haben jeweils einen mindestens zweiflankigen Hintergriff (43) zur spielfreien Aufnahme der Zylinder-Kolben-Einheit (100). Die einander gegenüber liegenden Flanken des Hintergriffs (43) schließen einen Winkel von z.B. 90 Winkelgraden ein. Die Länge und die Federrate der Federhaken (42) ist so dimensioniert, dass der Zylinder (101) ohne plastische Verformung der Federhaken (42) eingebaut werden kann.

Der Zylinder (101) ist z.B. ein klarsichtiger, dickwandiger Topf, dessen ggf. zylindrische Außenwandung eine beispielsweise umlaufende Rastrippe (102) trägt, die an den Flanken des Hintergriffs (43) der Federhaken (42) formsteif anliegt. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis
0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101). Diese Stirnfläche (103) kann zur Erhöhung der Applikationssicherheit zusätzlich mit einem Klebering (104) versehen werden.

Der Zylinder (101) ist an seinem dem Kolbenschieber (76) zugewandten Ende mit einer Abdichtfolie (119) steril verschlossen.

Zwischen dem Kolben (111) und dem Boden (39) ist der Federenergiespeicher (50) bzw. die Antriebseinheit des Einweginjektors angeordnet. Der Federenergiespeicher (50) ist eine Schraubendruckfeder, die auf dem Kolbenbetätigungsstempel (60) mit dem Stempelteller (73) angeordnet ist. Mittels des Stempeltellers (73) stützt sich der federkraftbelastete Kolbenbetätigungsstempel (60) an den Druckstäben (21) des Gehäuses (10) ab.

Der Kolbenbetätigungsstempel (60) hat oberhalb des Stempeltellers (73) einen Führungszapfen (62). Letzterer führt die Schraubendruckfeder (50). Unterhalb des Stempeltellers (73) befindet sich zentral in der Verlängerung des Führungszapfens (62) der Kolbenschieber (76), der bei einer Betätigung des Einmal-Injektors auf den Kolben (111) wirkt. Im Ausführungsbeispiel endet der Kolbenschieber (76) z.B. 2 bis 4 Millimeter oberhalb der Abdichtfolie (119) der Zylinder-Kolben-Einheit (100).

Im Gehäuse (10) ist nach Figur 5 am oberen Ende der Federhaken (42) eine gelochte Führungsscheibe (18) angeordnet. Sie sitzt dort z.B. eingeklemmt in einer Nut. Ggf. ist sie an dieser Stelle auch mit dem Gehäuse (10) verklebt. Die Führungsscheibe (18) zentriert den Kolbenschieber (76) vor dem Kolben (111) der Zylinder-Kolben-Einheit (100).

Das teilweise das Gehäuse (10) und die Zylinder-Kolben-Einheit (100) umgebende Auslöseelement (82) ist hier ebenfalls eine Auslösehülse. Die im Wesentlichen zylindrische, z.B. aus ABS gefertigte, Auslösehülse (82) hat an ihrem oberen Ende eine ringförmige radiale Aufweitung (83), die nach dem Auslösen des Einweginjektors die Nocken (22) der Druckstäbe (21) aufnimmt, vgl. Figuren 6 und 7. Die Aufweitung (83) wird gebildet durch eine Vielzahl von kurzen Federhaken (54). Hier gestalten z.B. 18 Federhaken (54) die Hüllfläche der Aufweitung, vgl. Figur 8.

Im unteren Bereich des Auslöseelements (82) befinden sich in dessen Außenwandung mehrere umlaufende Rillen (57) oder eine andere vergleichbare Struktur. Die Rillen (57) haben gegeneinander z.B. gleiche Abstände und erstrecken sich über 10 bis 30 Millimetern Länge des Auslöseelements (82).

An der unteren Stirnfläche (58) des Auslöseelements (82) liegt am Zylinder (101) der Zylinder-Kolben-Einheit (100) eine zentrierte Verschlusskappe (120) an. Ihre zumindest annähernd zylindrische Außenfläche hat den gleichen Durchmesser wie die ebenfalls zylindrische Außenfläche des Auslöseelements (82) in der Nähe der Stirnseite (58).

Die Verschlusskappe (120) ist ein Becher, der das untere Viertel der Zylinder-Kolben-Einheit (100) eng anliegend umgibt. Ein Teil der Verschlusskappe (120) umgibt mit ihrem Topfbereich (125) die zylindrische Außenwandung des Zylinders (101) und die untere Stirnfläche (103) mit dem dort befestigten Klebering (104). Im Zentrum des Topfbereiches (125) ist ein Hohlstopfen (127) ausgebildet, der die Ausnehmung (107) dicht verschließt. Der Topfbereich (125) selbst ist von einem Griffrohr (123) umgeben. Das Griffrohr (123) weist an seiner Außenwandung eine Rillung (124) oder andere Struktur auf.

Das zylindrische Auslöseelement (82) ist auf seiner gesamten Länge mit einem Klebeetikett (91) umhüllt. Das Klebeetikett (91) selbst ist z.B. ein mit einem Klebstoff bereichsweise einseitig beschichteter Papier- und/oder Folienstreifen. Der Folienstreifen umgibt z.B. einlagig einmal den Verbund aus Verschlusskappe (120) und Auslöseelement (82). Er besteht als Originalitätsverschluss (90) aus drei separaten Streifen, die jeweils über eine Perforation (96) gegeneinander abtrennbar sind. Der obere Streifen ist das Hauptteil (92), der mittlere Streifen ist eine Abreißbanderole (94) mit einer zwei bis drei Zentimeter langen Abreißfahne (95) und der untere Streifen ist das Kappenteil (93). Das Hauptteil (92) und das Kappenteil (93) tragen eine Klebeschicht, mit der sie an dem Auslöseelement (82) befestigt sind.

Zum Entsichern des Einweg-Injektors wird die Abreißbanderole (94) mit Hilfe der Abreißfahne (95) ringsherum vom Hauptteil (92) und vom Kappenteil (93) getrennt. Die Rillen (57) des Auslöseelements (82) werden sichtbar. Die Verschlusskappe (120) wird nun nach unten vom Zylinder (101) abgezogen.

Nun wird der Injektor auf die Injektionsstelle gesetzt und das hülsenartige Auslöseelement (82) nach unten - in Richtung der Injektionsstelle - geschoben. Hierbei rutschen die Nocken (22) über die Kante (85) nach außen in die Aufweitung (83). Die Druckstäbe (21) biegen sich elastisch nach außen in ihre eigentliche Ausgangslage. Die nun nicht mehr verformten Druckstäbe (21) geben den Kolbenbetätigungsstempel (60) frei, vgl. Figur 6, so dass sich der Kolben (111) unter der Wirkung des Federelements (50) ruckartig auf die Abdichtfolie (119) des Zylinder (101) zubewegt. Die Abdichtfolie (119) wird durchschlagen und der Kolben (111) zum Entleeren des Zylinders (101) nach unten bewegt, vgl. Figur 7.

Die Figuren 9 bis 14 stellen einen Druckstab-Injektor mit einer das Gehäuse fast vollständig umschließenden Auslöseeinheit (80) dar. An dem Auslöseelement (82) wird dazu eine Auslösekappe (81) befestigt, die das hintere Ende des Gehäuses (10) umgibt, vgl. auch Figur 4. Die Auslösekappe (81) ist dazu über das hintere Ende des Auslöseelements (82) geschoben. Dieses Ende hat als Stirnfläche die Rücksprungflanke (84) mit der innen liegenden Kante (85). Unmittelbar oberhalb der Rücksprungflanke (84) befindet sich in der Auslösekappe (81) die Aufweitung (83). Oberhalb der Aufweitung (83) liegt die Auslösekappe (81) gleitfähig an der Außenwandung (13) des Gehäuses (10) an.

Zur Befestigung der Auslösekappe (81) am Auslöseelement (82) hat das Auslöseelement (82) beispielsweise eine Ringnut (56), in die ein Umlaufsteg oder Rastnocken (55) der Auslösekappe (81) eingreifen. Nach den Figuren 9 und 11 bis 14 ist die Auslösekappe (81) - zur Erleichterung der Montage - z.B. zweifach bereichsweise längsgeschlitzt.

Am hinteren Ende hat die Auslösekappe (81) einen vertieft sitzenden Kappenboden (86). Am Kappenboden (86) sind um eine zentrische Bohrung herum z.B. mehrere nach innen ragende Rastzungen (87) angeformt. Die Rastzungen (87) weisen an ihren unteren Enden jeweils Zungenkerben (88) auf, die den Rand einer zentralen Bohrung (38) des Gehäusebodens (39) umgreifen.

Die Rastzungen (87) werden durch einen Sperrer (131) einer Druckknopfsicherung (130), vgl. Figur 10, in der den Boden (39) bereichsweise umgreifenden Position fixiert, so dass sich die Auslösekappe (81) in Kombination mit dem Auslöseelement (82) nicht gegenüber dem Gehäuse (10) in Längsrichtung bewegen kann.

Der Sperrer (131) hat einen elastischen, teilkugelschalenförmigen Sperrerknopf (132), an dem ein Sperrerbolzen (133) angeformt ist. Letzterer trägt an seinem unteren, freien Ende einen Blockierbund (134), der sich gegen eine Taille (135) absetzt. Der Blockierbund (134) hält die Rastzungen (87) in ihrer sperrenden Lage, vgl. Figur 10, und rastet hinter einem Raststeg (136) sicher ein.

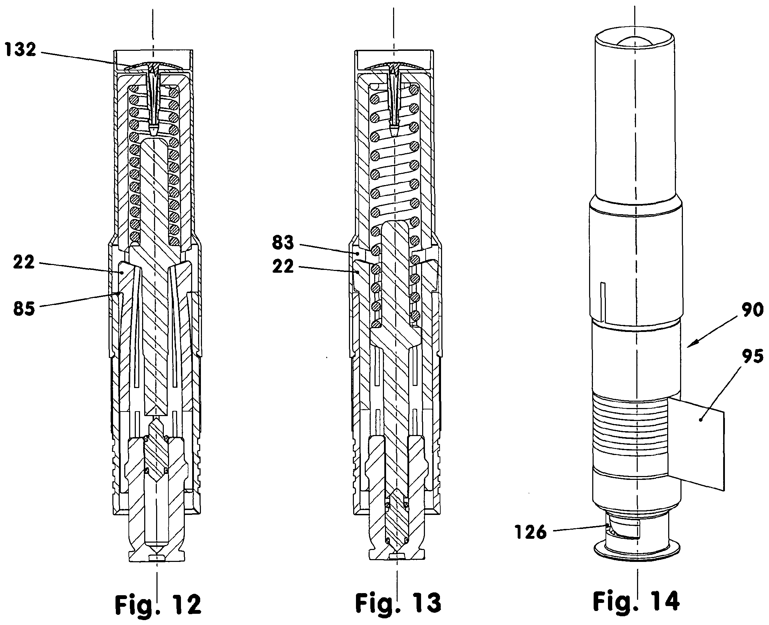

Wird der Sperrer (131) durch Niederdrücken betätigt, springen die federelastischen Rastzungen (87) hinter den Blockerbund (134) und legen sich an der Taille (135) an. Der Sperrer (131) verharrt dauerhaft in seiner betätigten Lage, vgl. Figuren 12 und 13. Die neue Hüllfläche der Rastzungen (87) hat nun einen Außendurchmesser, der kleiner ist als der Innendurchmesser der Bohrung (38). Folglich ist die mechanische Kopplung zwischen dem Auslöseelement (82) und dem Gehäuse (10) gelöst.

Um das Gehäuse (10) zusammen mit dem Federelement (50) und dem Kolbenbetätigungsstempel (60) bei der Montage im Auslöseelement (82) verliersicher fixieren zu können, hat das Gehäuse (10) in einem Bereich zwischen den Nocken (22) eine linsenförmige Erhebung (16), vgl. Figur 11, über die das Gehäuse (10) an der Kante (85) des Auslöseelements (82) anliegt.

Bei dem hier gezeigten Gehäuse (10) haben die Stützstäbe (21) Nocken (22) mit besonderen Hintergriffsflanken (25). Diese Hintergriffsflanken (25) liegen bei verformten Stützstäben (21) zumindest annähernd in einer zur Mittellinie (5) normalen Ebene. Demnach rasten sie beim Auslösen des Injektors schlagartig über die Kante (85). Nach dem Auslösen liegen sie zudem fest verrastet an der Rücksprungflanke (85) des Auslöseelements (82) an.

Der bei dieser Variante benutzte Kolbenbetätigungsstempel (60) hat einen Kolbenschieber (76) mit einer kegelmantelförmigen, nach innen gewölbten Stirnfläche (77), vgl. auch Figur 4. Mit dieser Stirnfläche (77) kontaktiert er die kegelige Spitze des Kolbens (111). Beide Kegel haben zumindest annähernd den gleichen Kegelwinkel. Der dargestellte Kolben (111) ist Gegenstand des Patentes DE 10 2006 045 959 C1.

Die Verschlusskappe (120) nach den Figuren 9 bis 12 umhüllt nicht nur bereichsweise den Zylinder (101) und liegt dabei an dem Auslöseelement (82) an, sondern stützt sich zusätzlich am Gehäuse (10) ab. Dazu hat sie in der Nähe der oberen kegelstumpfmantelförmigen Stirnfläche (121) mehrere an der Innenwandung angeordnete Anlagestege (122). Letztere sind parallel zur Mittellinie (5) ausgerichtet. Die Anlagestege (122) kontaktieren die Federhaken (42).

Der Topfbereich (125) weist zwei einander gegenüberliegende Fenster (126) auf. Die Fenster (126) haben eine Breite, die mindestens dem Durchmesser des Kolbens (111) entspricht. Die Unterkante der Fenster (126) - also die Kanten, die dem tellerartigen Fuß (128) an nächsten liegen - sind in der Höhe des Zylinderbodens (108) angeordnet. Mit Hilfe der Fenster (126) lässt sich im Durchlicht u.a. die Blasenfreiheit der Zylinderbefüllung prüfen.

Bei dieser Ausführungsvariante können, mit Ausnahme des Federelements (50), alle Bauteile rotationssymmetrisch und/oder zu einer auf der Mittellinie (5) gelegenen Ebene spiegelsymmetrisch aufgebaut sein.

Das Benutzen dieses Injektors entspricht zumindest weitgehend der Benutzung der zuvor beschriebenen Injektorvariante. Allerdings wird hier das zusätzliche Sicherungselement (130) verwendet. Nach dem Abziehen der Abreißbanderole (94) und dem Entfernen der Verschlusskappe (120) bleibt der Injektor gesichert. Nach dem Aufsetzen des Injektors auf die Injektionsstelle muss z.B. mit dem Daumen der den Injektor haltenden Hand der Sperrerknopf (132) gedrückt werden, um das Auslöseelement (82) zusammen mit der Auslösekappe (81) bewegen zu können.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Bei den in den Figuren dargestellten Varianten ist die einzelne Kontaktzone zwischen dem Druckstab (21) und dem Stempelteller (73), als Flächen (23) und (74, 75) ausgeführt, die gleitfähig einander kontaktieren. In einer besonderen Ausgestaltung kann in jeder Fläche (23) der einzelnen Druckstäbe (21) eine Walze gelagert werden, die bei einer Betätigung des Injektors an der Fläche (74, 75) des Stempeltellers wälzgelagert, also reibungsarm, abrollt.

Mit Ausnahme des Federelements (50), einer ggf. vorhandenen Kolbenplatte und der beispielsweise vorhandenen Lagerwalzen der Stützstäbe (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil
- 8: Sperrstellung
- 9: Lösestellung, Auslösestellung

- 10: Gehäuse, einteilig
- 13: Außenfläche, zylindrisch
- 16: Erhebung, linsenförmig
- 18: Führungsscheibe

- 21: Druckstäbe, Stützstäbe
- 22: Nocken
- 23: Abstützfläche
- 24: Anlagefläche
- 25: Hintergriffsflanke
- 28: Biegebalken

- 31: Mantelbereich
- 33: Durchbrüche
- 38: Bohrung
- 39: Boden

- 41: Fixierbereich für die Zylinder-Kolben-Einheit
- 42: Federhaken
- 43: Hintergriff

- 50: Federelement, Schraubendruckfeder, Federenergiespeicher
- 52: Federelemente an (21)
- 54: Federhaken, kurz
- 55: Rastnocken
- 56: Ringnut von (82)
- 57: Rillen von (82)
- 58: Stirnfläche von (82)
- 59: Innenwandung von (82)

- 60: Kolbenbetätigungsstempel
- 62: Führungszapfen

- 73: Stempelteller
- 74: Bundfläche, plan
- 75: Bundfläche, konisch
- 76: Kolbenschieber
- 77: Kolbenschieberstirnfläche, kegelmantelförmig
- 79: Unterlagscheibe

- 80: Auslöseeinheit
- 81: Auslösekappe
- 82: Auslöseelement
- 83: Aufweitung
- 84: Rücksprungflanke
- 85: Kante, scharfkantig
- 86: Kappenboden
- 87: Rastzungen
- 88: Zungenkerbe

- 90: Originalitätsverschluss, Banderole, Sicherungselement
- 91: Klebeetikett
- 92: Hauptteil von (91)
- 93: Kappenteil von (91)
- 94: Abreißbanderole
- 95: Abreißfahne
- 96: Perforationen, Sollbruchstellen

- 100: Zylinder-Kolben-Einheit
- 101: Zylinder
- 102: Rastrippe
- 103: Stirnfläche
- 104: Klebering
- 106: Bohrung, Düse
- 107: Ausnehmung in der Stirnfläche
- 108: Zylinderboden
- 111: Kolben
- 112: Ringnut
- 114: Dichtring, Dichtung
- 119: Abdichtfolie

- 120: Verschlusskappe, Klebeversiegelung
- 121: Stirnfläche, oben
- 122: Anlagestege
- 123: Griffrohr
- 124: Struktur, Rillung
- 125: Topfbereich
- 126: Fenster, beidseitig
- 127: Hohlstopfen
- 128: Fuß

- 130: Druckknopfsicherung, Sicherungselement
- 131: Sperrer
- 132: Sperrerknopf
- 133: Sperrerbolzen
- 134: Blockierbund
- 135: Taille
- 136: Raststeg

## Patentansprüche

1. Einweginjektor mit einem Gehäuse (10), in dem oder an dem - jeweils zumindest bereichsweise - mindestens ein mechanischer Federenergiespeicher, mindestens eine - zumindest zeitweise wirkstoffbefüllbare - Zylinder-Kolben-Einheit (100), mindestens ein Kolbenbetätigungsstempel (60) und mindestens eine Auslöseeinheit (80) angeordnet ist, wobei der Federenergiespeicher (50) mindestens ein vorgespanntes Federelement umfasst und wobei zumindest ein einen Stempelteller (73) bildender Teil des Kolbenbetätigungsstempels (60) zwischen dem Federenergiespeicher (50) und dem Kolben (111) der Zylinder-Kolben-Einheit (100) positioniert ist, sodass das Federelement proximal auf dem Stempelteller (73) angeordnet ist,
- wobei das Gehäuse (10) mindestens einen Druckhaken (21) aufweist, der jeweils im Bereich seines freien Endes mindestens eine Abstützfläche (23) hat,
- wobei die sperrende Lage des Druckhakens (21) durch ein in einer Sperrstellung (8) positioniertes Auslöseelement (82) gesichert ist und
- wobei das Auslöseelement (82) eine Sperrstellung (8) hat, in der es an einer Verschlusskappe (120) gesichert anliegt und zum Auslösen auf einer Außenwandung (13) des Gehäuses (10) linear nach unten, in Richtung der Injektionsstelle, gleitet,
- wobei vor Betätigung des Einweginjektors die Verschlusskappe (120) den unteren - mit einer Düse (106) ausgestatteten - Bereich des Zylinders (101) der Zylinder-Kolben-Einheit (100) umgibt, **dadurch gekennzeichnet,**
- **dass** an der Abstützfläche (23) der Kolbenbetätigungsstempel (60) mit seinem zumindest bereichsweise als zylindrische Scheibe ausgebildeten Stempelteller (73) anliegt, sodass sich der federkraftbeaufschlagte Stempelteller distal auf dem Druckhaken abstützt, und
- **dass** das Auslöseelement (82) eine Lösestellung (9) hat, die ein seitliches Zurückweichen des Druckhakens (21) - unter Freigabe des Kolbenbetätigungsstempels (60) - bewirkt, sodass der Kolbenbetätigungsstempel (60) ungehindert nach unten schnellt.

2. Einweginjektor gemäß Anspruch 1, **dadurch gekennzeichnet, dass** der Kolbenbetätigungsstempel (60) an seiner - dem Federelement (50) abgewandten - Stirnfläche zumindest bereichsweise ebene Keilflächen oder bereichsweise einzelne kegelstumpfmantelförmige Flächen (74, 75) aufweist.

3. Einweginjektor gemäß Anspruch 1, **dadurch gekennzeichnet, dass** der Kolbenbetätigungsstempel (60) zusammen mit jedem einzelnen Stützstab (21) ein Schiebekeilgetriebe bildet, in dem eine axiale Federkraftrichtung in eine radiale Stützkraftrichtung umgelenkt wird.

4. Einweginjektor gemäß Anspruch 1, **dadurch gekennzeichnet, dass** der einzelne Stützstab (21) jeweils am Gehäuse (10) angeformt ist und einen elastischen Biegebalken (28) darstellt.

5. Einweginjektor gemäß Anspruch 1, **dadurch gekennzeichnet, dass** der einzelne Stützstab (21) an seinem freien Ende eine ebene, kegelstumpfmantelförmige oder sphärische Abstützfläche (23) aufweist.

6. Einweginjektor gemäß Anspruch 1, **dadurch gekennzeichnet, dass** die Verschlusskappe (120) bündig an der unteren Stirnseite des Auslöseelements (82) anliegt.

7. Einweginjektor gemäß Anspruch 1, **dadurch gekennzeichnet, dass** die Verschlusskappe (120) am Zylinder (101) der Zylinder-Kolben-Einheit (100) zentriert ist.

8. Einweginjektor gemäß Anspruch 1, **dadurch gekennzeichnet, dass** die Verschlusskappe (120) im unteren Bereich zwei einander gegenüberliegende Fenster (126) aufweist, deren Mittellinien eine Mittellinie (5) des Injektors schneiden oder zumindest im Abstand von 0,01 bis 5 Millimeter kreuzen.

9. Einweginjektor gemäß Anspruch 8, **dadurch gekennzeichnet, dass** die Fenster (126) eine quer zur Mittellinie (5) orientierte Breite haben, die mindestens dem Durchmesser des Kolbens (111) der Zylinder-Kolben-Einheit (100) entspricht.

10. Einweginjektor gemäß Anspruch 1, **dadurch gekennzeichnet, dass** das Auslöseelement (82) in Kombination mit der Verschlusskappe (120) und einem an ihm befestigten Originalitätsverschluss (90) eine gesicherte Auslöseeinheit (80) bildet.

11. Einweginjektor gemäß Anspruch 10, **dadurch gekennzeichnet, dass** der Originalitätsverschluss (90) ein Klebeetikett (91) ist, das eine Abreißbanderole (94) umfasst, die ein im inneren Bereich des Klebeetiketts (91) angeordneter Steifen ist, der gegenüber dem restlichen Bereich des Klebeetiketts (91) über eine Perforation (96) verbunden ist.

12. Einweginjektor gemäß Anspruch 11, **dadurch gekennzeichnet, dass** die Abreißbanderole (94) eine zwischen dem Auslöseelement (82) und der Verschlusskappe (120) gelegenen Montagefuge überdeckt oder an dieser angrenzt.

## Claims

1. A disposable injector comprising a housing (10) having disposed at least section-wise therein or thereon at least one mechanical spring energy storage means, at least one cylinder-piston unit (100) adapted to be filled at least temporarily with an active agent, at least one piston actuating plunger (60) and at least one release unit, said spring energy storage means (50) comprising at least one biased spring element and at least one part forming a plunger disk (73) of the piston actuating plunger (60) and positioned between the spring energy storage means (50) and the piston (111) of the piston-cylinder unit (100) so that said spring element is located proximally on the plunger disk (73),
- the housing (10) comprising at least one pressure hook element (21) each having at least one support surface (23) in the area of its free end,
- with the pressure hook element (21) being secured in its locked position by a release element (82) positioned in a locking position (8),
- with the release element (82) having a locking position (8) in which it is secured to a closure cap (120) and is released from it to slide linearly downwards along an outer wall (13) of the housing (10) towards the injection site, and
- with the closure cap (120) surrounding the bottom region of the cylinder (101) of the cylinder-piston unit (100) prior to actuation of the disposable injector, said bottom region being provided with an orifice (106),
**characterized in that**
- the plunger disk (73) engages the support surface (23) of the piston actuating plunger (60) by its plunger disk (73), which is formed at least section-wise as a cylindrical disk, so that the spring-force-actuated plunger disk distally rests on said pressure hook, and **in that**
- the release element (82) has a release position (9) in which the pressure hook (21) is made to recede and release the piston actuating plunger (60) so that the latter snaps downwards unchecked.

2. Disposable injector as claimed in claim 1, **characterized in that** the piston actuating plunger (60) has on its face turned away from spring element (50) wedge areas that are planar at least section-wise or detached areas (74, 75) that are peripherally frustroconical section-wise.

3. Disposable injector as claimed in claim 1, **characterized in that** the piston actuating plunger (60) forms together with each individual supporting rod (21) a wedge-displacement-type transmission assembly in which an axial spring force is redirected to generate a radial supporting force.

4. Disposable injector as claimed in claim 1, **characterized in that** each individual supporting rod (21) is integral with the housing (10) and constitutes a resilient flexing beam (28).

5. Disposable injector as claimed in claim 1, **characterized in that** each individual supporting rod (21) has at its free end a planar frustroconical or spherical engagement surface (23).

6. Disposable injector a claimed in claim 1, **characterized in that** the closure cap (120) is flush with the bottom face of the release element (82).

7. Disposable injector as claimed in claim 1, **characterized in that** the closure cap (120) is centered on the cylinder (101) of the cylinder-piston-unit (100).

8. Disposable injector as claimed in claim 1, **characterized in that** the closure cap (120) has in its bottom area a pair of opposing windows (126) of which the center lines intersect on the center line (5) of the injector or at least cross said center line (5) at a distance of 0.01 mm to 5 mm.

9. Disposable injector as claimed in claim 8, **characterized in that** the windows (126) have a width transverse to the center line (5) corresponding to at least the diameter of the piston (111) of the cylinder-piston-unit (100).

10. Disposable injector as claimed in claim 1, **characterized in that** the release element (82) is configured in combination with the closure cap (120) and a tamper-proof closure (90) to form a secured release unit (80).

11. Disposable injector as claimed in claim 10, **characterized in that** the tamper-proof closure (90) is a stick-on label (91) comprising a section of tear-off band stock (94) in the form of a strip disposed in the inner region of the stick-on label (91), said strip being connected with the remainder of the stick-on label (91) via perforations (96).

12. Disposable injector as claimed in claim 11, **characterized in that** the tear-off band stock (94) covers, or is contiguous, with an assembly joint situated between the release element (82) and the closure cap (120).

## Revendications

1. Injecteur à usage unique, comprenant un boîtier (10) dans lequel ou sur lequel sont disposés, en tout cas par endroits, au moins un accumulateur d'énergie à ressort mécanique, au moins une unité cylindre-piston (100), pouvant être remplie d'une substance active au moins temporairement, au moins un poinçon d'actionnement de piston (60) et au moins une unité de déclenchement (80), l'accumulateur d'énergie à ressort (50) comprenant au moins un élément de ressort précontraint et au moins une partie du poinçon d'actionnement de piston (60), qui forme une coupelle (73), étant positionnée entre l'accumulateur d'énergie à ressort (50) et le piston (111) de l'unité cylindre-piston (100), de telle façon que l'élément de ressort est placé en position proximale sur la coupelle (73) du poinçon d'actionnement,
- le boîtier (10) présentant au moins un crochet de pression (21) qui possède au moins une surface d'appui (23) au niveau de son extrémité libre,
- la position de blocage du crochet de pression (21) étant fixée par un élément de déclenchement (82) positionné dans une position de verrouillage (8) et
- l'élément de déclenchement (82) présentant une position de verrouillage (8), dans laquelle il s'applique de manière fixée contre un capuchon de fermeture (120) et dans laquelle, à des fins de déclenchement, il glisse sur une paroi extérieure (13) du boîtier (10) de façon linéaire vers le bas, en direction du point d'injection,
- avant l'actionnement de l'injecteur à usage unique, le capuchon de fermeture (120) entourant la partie inférieure du cylindre (101) équipée d'une buse (106) de l'unité cylindre-piston (100), **caractérisé en ce**
- **que** le poinçon d'actionnement de piston (60) repose avec la coupelle (73), qui est réalisée au moins en partie sous forme d'un disque cylindrique, sur la surface d'appui (23) de telle façon que la coupelle sollicitée par la force d'un ressort vient s'appuyer de façon distale sur le crochet de pression,
- **que** l'élément de déclenchement (82) présente une position de libération (9) qui provoque un recul latéral du crochet de pression (21) en libérant le poinçon d'actionnement de piston (60) de manière à permettre au poinçon d'actionnement de piston (60) de se déplacer librement vers le bas.

2. Injecteur à usage unique selon la revendication 1, **caractérisé en ce que** le poinçon d'actionnement de piston (60) présente, au niveau de sa surface frontale opposée à l'élément de ressort (50), au moins en partie des surfaces de clavette planes ou en partie des surfaces en forme d'enveloppe tronconique individuelles (74, 75).

3. Injecteur à usage unique selon la revendication 1, **caractérisé en ce que** le poinçon d'actionnement de piston (60) forme avec chaque tige de support (21) un mécanisme à cale coulissante dans lequel une direction de force de ressort axiale est déviée dans une direction de force de support radiale.

4. Injecteur à usage unique selon la revendication 1, **caractérisé en ce que** chaque tige de support (21) est façonnée sur le boîtier (10) et forme une barre de flexion élastique (28).

5. Injecteur à usage unique selon la revendication 1, **caractérisé en ce que** chaque tige de support (21) présente à son extrémité libre une surface d'appui (23) plane en forme d'enveloppe tronconique ou une surface d'appui sphérique.

6. Injecteur à usage unique selon la revendication 1, **caractérisé en ce que** le capuchon de fermeture (120) est en affleurement avec la face frontale inférieure de l'élément de déclenchement (82).

7. Injecteur à usage unique selon la revendication 1, **caractérisé en ce que** le capuchon de fermeture (120) est centré sur le cylindre (101) de l'unité cylindre-piston (100).

8. Injecteur à usage unique selon la revendication 1, **caractérisé en ce que** le capuchon de fermeture (120) présente dans sa partie inférieure deux fenêtres (126) opposées l'une par rapport à l'autre dont les lignes médianes coupent une ligne médiane (5) de l'injecteur ou au moins se croisent dans une distance de 0,01 à 5 millimètres.

9. Injecteur à usage unique selon la revendication 8, **caractérisé en ce que** les fenêtres (126) ont une largeur qui est orientée transversalement à la ligne médiane et qui correspond au moins au diamètre du piston (111) de l'unité cylindre-piston (100).

10. Injecteur à usage unique selon la revendication 1, **caractérisé en ce que** l'élément de déclenchement (82) en combinaison avec le capuchon de fermeture (120) et une fermeture inviolable (90) fixée sur l'élément de déclenchement forment une unité de déclenchement sécurisée (80).

11. Injecteur à usage unique selon la revendication 10, **caractérisé en ce que** la fermeture inviolable (90) est une étiquette adhésive (91) comprenant une bande de déchirage (94) qui est une bande disposée dans la partie intérieure de l'étiquette adhésive (91) et reliée au reste de l'étiquette adhésive (91) par une perforation (96).

12. Injecteur à usage unique selon la revendication 11, **caractérisé en ce que** la bande de déchirage (94) couvre un joint de montage ou est adjacente à un joint de montage situé entre l'élément de déclenchement (82) et le capuchon de fermeture (120).
